Europäisches Patentamt

European Patent Office

Office européen des brevets

(19)

(11) EP 0 840 597 B1

(12) EUROPEAN PATENT SPECIFICATION

(45) Date of publication and mention
of the grant of the patent:
01.10.2003  Bulletin 2003/40

(51) Int Cl.⁷: **A61K 9/20**, C12Q 1/00,
A61F 13/02

(21) Application number: 96924493.8

(22) Date of filing: 11.07.1996

(86) International application number:
PCT/US96/11675

(87) International publication number:
WO 97/002811 (30.01.1997 Gazette 1997/06)

(54) **HYDROGEL PATCH**

HYDROGELPFLASTER

PASTILLE D'HYDROGEL

(84) Designated Contracting States:
**AT BE CH DE DK ES FI FR GB GR IE IT LI LU MC NL PT SE**

(30) Priority: 12.07.1995  US 501664

(43) Date of publication of application:
13.05.1998  Bulletin 1998/20

(73) Proprietor: **CYGNUS, INC.**
**Redwood City, CA 94063 (US)**

(72) Inventors:
• **ABRAHAM, William**
**Fremont, CA 94536 (US)**
• **BERNER, Bret**
**El Granada, CA 94018 (US)**
• **JOSHI, Priti, S.**
**San Jose, CA 95129 (US)**
• **PLANTE, Phillip, J.**
**Sunnyvale, CA 94086 (US)**
• **VIJAYAKUMAR, Prema**
**Fremont, CA 94539 (US)**

(74) Representative: **Hallybone, Huw George et al**
**Carpmaels and Ransford,**
**43 Bloomsbury Square**
**London WC1A 2RA (GB)**

(56) References cited:
**WO-A-96/00110**

• **BIOMATERIALS, vol. 15, no. 7, 1994, NEW YORK NY USA, pages 502-506, XP000611255 H.A. ALLOCK ET AL.: "Activity of urea amidohydrolase immobilized within poly(di(methoxyethoxyethoxy)phosphazene) hydrogels"**
• **JOURNAL OF PHAMACEUTICAL SCIENCES, vol. 68, no. 7, 1 July 1979, WASHINGTON DC USA, pages 919-921, XP000611218 J. HELLER ET AL.: "Controlled drug release by polymer dissolution II: enzyme-mediated deleivery device"**
• **JOURNAL OF BIOMEDICAL MATERIALS RESEARCH, vol. 19, no. 9, 1985, NEW YORK NY USA, pages 1117-1133, XP000609881 J. KOST ET AL.: "Glucose-sensitive membranes containing glucose oxidase: activity, swelling and permeability studies"**
• **ARTIFICIAL CELLS, BLOOD SUBSTITUTES AND IMMOBILIZATION BIOTECHNOLOGY, vol. 23, no. 5, 1 May 1995, WASHINGTON DC USA, pages 587-595, XP000609870 E.M. D'URSO ET AL.: "poly(ethylene glycol)-serum albumin hydrogel as matrix for enzyme immobilization: biomedical applications"**

## EP 0 840 597 B1

**Description**

Field of the Invention

This invention relates generally to the field of hydrogels which contain components which enhance the performance of the gel for a particular purpose including hydrogel patches used in the medical fields.

Background of the Invention

There are a number of known hydrophilic, polymeric compounds which form various cellular groups and/or networks creating a gel in the presence of water. For example, gelatin can be obtained by the hydrolysis of collagen by boiling skin, ligaments, tendons, etc. A mixture of only 2% gelatin in water will form a stiff gel.

A hydrogel may be formed by adding a solute such as gelatin to water at an elevated temperature to dissolve gelatin. The solution is then cooled and the solute(s) (e.g., solid gelatin components) form submicroscopic crystalline particle groups which retain a great deal of solvent (generally water) in the interstices (so-called "brush-heap" structure). Gels, and in particular hydrogels, are usually transparent but may be opalescent.

Gels may be formed from naturally occurring or synthetic materials and have a wide range of uses including photographic film; sizing; textile and paper adhesives; cements; capsules and patches for medicinals; matches; light filters; desserts; culture medium for bacteria; and patches used with electronic medical monitoring equipment.

Gels generally contain a very high concentration of water, e.g., about 60% to about 98% water and are held together by a variety of cellular groups. The water may be bound or unbound- form various hydrates with the solute or be entrapped in cellular pockets formed by the polymer network groups. Although gels have some general features in common they have such diverse uses that it is necessary to modify the components being included to obtain a desired result. For example, flavoring would be added to a dessert gelatin but not a light filter gelatin. However, coloring agents might be added to either although the coloring agent might be very different for desserts than for light filters.

Various prior art documents disclose hydrogels containing enzymes, for example H.R. Allcock et al., Biomaterials, vol. 15, no. 7, pages 502-506, 1994; J. Heller et al., American Pharmaceutical Association, vol. 68, no. 7, pages 919-921, 1979; J. Kost et al., Journal of Biomedical Materials Research, vol. 19, pages 1117-1133, 1985; and E.M. D'Urso et al., Artificial Cells, Blood Substitutes and Immobilization Biotechnology, vol. 23, no. 5, pages 587-595, 1995. However, none of the hydrogels disclosed in the prior art has the characteristics which the hydrogels of the present invention have.

The hydrogel patch of the invention has been designed to include very specific components in specific amounts so that the desired end results are obtained.

Summary of the Invention

A patch is disclosed which is comprised of a hydrophilic compound which forms a material which holds water in place and allows the flow of electrical current therethrough. The compound may be an absorbent material, porous material or polymers which may be cross-linked to form a porous network of interconnected cells or a solute which forms a gel with water. The solute or solid material component of the gel is generally present in an amount of about 0.5% or more and preferably less than 40% by weight based on the weight of the patch. The water, and the patch as a whole, is made electrically conductive by the inclusion of a chloride containing salt such as NaCl. The patch comprises an enzyme, which catalyzes a reaction such as a reaction with glucose allowing for the formation of hydrogen peroxide in water and ultimately generating the release of two electrons per molecule of glucose. Glucose drawn into the patch is reduced to gluconic acid and hydrogen peroxide with the aid of the enzyme and in use resulting in electrons being released which can be detected and related to the amount of glucose entering the patch. The patch is also preferably comprised of a buffer which maintains the pH of the patch in the range of from about 3 to 9, and may be further comprised of a cross-linking agent, a biocide, a humectant and, a surfactant. The patch is preferably in the form of a thin, flat disc which will conform to the contours of human skin and may have a non-woven fabric or porous membrane (for example, nitrocellulose) embedded therein.

An object of the invention is to provide a disposable device which proportionally converts a biologically important molecule such as glucose entering the device to predetermined amounts of a detectable signal such as current which can be measured.

Another object is to provide a hydrogel patch which is comprised of a gel forming compound and water along with glucose oxidase and a chloride containing salt which renders the gel electrically conductive.

An advantage of the invention is that it makes it possible to continuously and accurately measure an inflow of a very small amount of glucose e.g., concentrations 10, 500 or 1,000 or more times less than the concentration of glucose in blood.

Another advantage is that background electrical signal ("noise", signal in the absence of analyte) is low relative to signal in the presence of analyte. In a preferred embodiment of the invention, the background noise is less than about 200 nanoAmperes (nA), preferably less than about 50 nA.

Another advantage of one embodiment of the invention is the stability of peroxide in the gel. Preferably, loss of peroxide, independent of the glucose oxidase reaction, is less than about 20% over a period of 30 minutes.

Another advantage of the invention is that the water loss from the gel is less than about 70% over a 24 hour time period.

Another advantage of the invention is that the speed of analyte transport through the gel is rapid relative to the interval of time over which a measurement is taken ($t_m$, measurement time for the analyte). Transport is related to the characteristic time of the gel. The term "characteristic time for a gel" is used herein to refer to analyte diffusion-related function of the gel which is, in turn, related to the thickness of the gel (L, the distance the analyte diffuses) and the diffusion constant of the analyte (D). The relationship between the parameters L and D is the following:

$$L^2/D = \text{Characteristic time, minutes}$$

Preferably, the characteristic time of a gel of the invention is approximately 6 seconds to 45 minutes. Preferably, a measurement of analyte in the gel is integrated over a desired period of time at a desired time interval (such as over a 5 minute period, measured every 20 minutes). From the above parameters, the transport of analyte in the gel is defined by the ratio of measurement time to the characteristic time:

$$[D \times t_m]/L^2 > 1$$

where D, L and $t_m$ are defined above.

Another advantage is that the patch is easily and economically produced and is disposable.

A feature of the hydrogel patch is that it is flat and thin having a surface area in the range of about 0.5 cm$^2$ to 10 cm$^2$ and a thickness in the range of about 0.025 mm (1 mil) to about 1.27mm (50 mils).

Another feature of the invention is that the hydrogel patch is further comprised of a structural support such as a non-woven fabric or filaments or structural support membrane embedded in the patch.

Yet another feature of the invention is that the gel forming material may be cross-linked by the application of ionizing radiation such as electron beam radiation, UV light, heat or the use of association coupling which cross-linking may be facilitated by the addition of a cross-linking agent.

These and other objects, advantages and features of the present invention will become apparent to those persons skilled in the art upon reading the details of the composition, components and size of the invention as set forth below reference being made to the accompanying drawings forming a part hereof wherein like numbers refer to like components throughout.

Brief Description of the Drawing

Fig. 1 is a cross-sectional schematic view of the hydrogel patch of the invention;
Fig. 2 is a overhead schematic view of the hydrogel patch of the invention;
Fig. 3 is a cross-sectional schematic view of an alternative embodiment of the invention;
Fig. 4 is a schematic representation of the reaction which glucose oxidase (GOX) catalyzes in order to obtain gluconic acid and hydrogen peroxide and result in the generation of current; and
Fig. 5 is a graph showing the relationship between the concentration of the enzyme within the patch and the electrical signal generated as a result of a reaction catalyzed by the enzyme.

Description of the Embodiments

Before the patch of the present invention is described and disclosed it is to be understood that this invention is not limited to the particular components or amounts described as such may, of course, vary. It is also to be understood that the terminology used herein is for the purpose of describing particular embodiments only, and is not intended to be limiting since the scope of the present invention will be limited only by the appended claims.

It must be noted that as used in this specification and the appended claims, the singular forms "a", "an" and "the" include plural referents unless the context clearly dictates otherwise. Thus, for example, reference to "a salt" includes a plurality of salt molecules and different types of salts, reference to "an enzyme" refers to a plurality of enzyme molecules and so forth.

Unless defined otherwise all technical and scientific terms used herein have the same meaning as commonly understood by one of ordinary skill in the art to which this invention belongs. Although any materials or methods similar or equivalent to those described herein can be used in the practice or testing of the present invention, the preferred methods and materials are now described. All publications mentioned herein are incorporated herein by reference for the purpose of describing and disclosing the particular information for which the publication was cited in connection with.

Definitions

The terms "hydrogel", "gel" and the like, are used interchangeably herein to refer to a material which is not a readily flowable liquid and not a solid but a gel which gel is comprised of from 0.5% or more and preferably less than 40% by weight of gel forming solute material and from 95% or less and preferably more than 55% water. The gels of the invention are preferably formed by the use of a solute which is preferably a synthetic solute (but could be a natural solute, e.g., for forming gelatin) which forms interconnected cells which binds to, entrap, absorb and/or otherwise hold water and thereby create a gel in combination with water,
where water includes bound and unbound water. The gel is the basic structure of the hydrogel patch of the invention will include additional components beyond the gel forming solute material and water such as an enzyme and a salt which components are further defined below.

The terms "gel forming material", "solute" and the like are used interchangeably herein to refer to a solid material which, when combined with water, forms a gel which gel, in general, is created by the formation of any structure which holds water including interconnected cells and/or network structure formed by the solute. The solute may be a naturally occurring material such as the solute of natural gelatin which includes a mixture of proteins obtained by the hydrolysis of collagen by boiling skin, ligaments, tendons and the like. However, the solute or gel forming material is more preferably a polymer material (including, but not limited to, polyethylene oxide, polyvinyl alcohol, polyacrylic acid, polyacrylamidomethylpropanesulfonate and copolymers thereof, and polyvinyl pyrrolidone) present in an amount in the range of more than 0.5% and less than 40% by weight, preferably 8 to 12% by weight when a humectant is also added, and preferably about 15 to 20% by weight when no humectant is added. The solid material may include additional components such as polyacrylic acid present in an amount in the range of 0.5 to 5% by weight and more preferably about 2% by weight which polyacrylic acid is sold under the trade name Carbopol®. Preferably, the gel forming material or any component of the gel does not react with the solute or its detectable reaction product such that measurement and quantitation is adversely affected. For example, polyvinyl pyrrolidone was observed to react with hydrogen peroxide, and is therefore, not a preferred gel forming material for use in detecting glucose via the glucose oxidase reaction where hydrogen peroxide is the compound being measured

The gel forming material may, for example, include a cross-linked polymeric material which forms a gel as described above or a naturally occurring or synthetic sponge which absorbs water. The material may hold the water by partially encapsulating the water in cellular units or be a fibrous paper-like material which holds the water by capillary action. Preferred materials can hold an amount of water which is equal to or greater than the amount of solid material based on the weight of the water and material. More preferably, the material holds an amount of water which is greater than approximately 2 to 5, most preferably greater than about 15 times the weight of the material.

The term "water loss" is used herein to refer a measurement of the rate of water loss over a specified period of time. For optimal function of the gel, it is preferred that water loss is less than 70% over a 24 hour period. Water loss is measured as follows: The gel, approximately 1.9cm (0.75 inches) in diameter, was placed between circular disks such that water vapor could escape only from the sides of the gel. Weight loss was measured at selected time points over a period of 24 hours at ambient temperature and pressure. Weight loss was attributed to water loss, and was normalized to the initial water content of the gel. A gel drying rate of less than 70% over 24 hours was preferred. Humectants may be added to the gel mixture to improve water retention properties of the gel.

The term "buffer" is used herein to refer to the components added to the water of the patch or gel in order to maintain the pH within a defined range. The buffer includes a weak acid and its conjugate weak base whose pH changes only slightly on the addition of acid or alkaline. The weak acid becomes a buffer when alkali is added and the weak base becomes a buffer on addition of acid. This buffering action is explained by the reaction

$$A + H_2O \rightarrow B^- + nH_3O^+$$

wherein n is a positive integer and $B^-$ is a weak base and A is a weak acid. The base B is formed by the loss of a proton from the corresponding acid A. The acid may contain cations such as $NH^{4+}$, a neutral molecule such as $CH_3COOH$, or an anion such as $H_2PO^-_4$. When alkali is added, hydrogen ions are removed to form water, but, as long as the added alkali is not in excess of the buffer acid, many of the hydrogen ions are replaced by further ionization of A to maintain the equilibrium. When acid is added, this reaction is reversed as hydrogen ions combined with the base B to form the acid A. A variety of different buffers can be used in connection with the present invention including, but not limited to phosphate buffer and bicarbonate salt present in amounts sufficient to maintain the pH of the hydrogel in a range of about 3 - 9, more preferably 6 - 8.

The terms "salt" and "chloride salt" are used interchangeably herein to describe a chloride containing compound formed when the hydrogen of an acid is replaced by a metal or its equivalent. For example,

$$HCl + NaOH \rightarrow NaCl + H_2O.$$

Salts useful in connection with the present invention are added to the water component in an amount sufficient to provide for electrical conductivity of the patch. The salt is preferably present in an amount in the range of from about 0.1% to about 5% preferably 0.3% to 2% by weight based on the weight of the hydrogel. The salts preferably contain a chloride ion. Preferred salts include sodium chloride, potassium chloride and magnesium chloride with NaCl being most preferred.

The term "humectant" is used herein to describe a substance which has an affinity for water and a stabilizing action on the water content of the gel material.

The terms "cross-linker" and "cross-linking agent" are used herein to describe compounds which is combined with polymers to facilitate cross-linking which may be initiated by irradiation (e.g., U.V., e-beam, etc.), thermal or chemical means. Cross-linking can be enhanced by the addition of a cross-linking agent where the polymer or polymers are subjected to radiation such as electron beam radiation, ionization radiation, gamma radiation, or U.V. light which activates groups on a polymer backbone or pendant moiety and allows the activated groups to bind with other groups on another polymer chain. Cross-linking improves the structural integrity of the patch.

The term "biocide" is used herein to describe any substance that kills or inhibits the growth of microorganisms such as bacteria, molds, slimes, fungi, etc. A biocide may be a material which is also toxic to humans but is preferably a material which, when used in relatively low concentrations in a patch or the hydrogel does not cause skin irritation or any adverse effects on a human patient. Biocide chemicals include compounds such as chlorinated hydrocarbons, organometallics, hydrogen releasing compounds, metallic salts, organic sulfur compounds, quaternary ammonium compounds, phenolics, methyl parabens and the like. If a biocide compound is used in connection with the present invention the amount is less than 0.5% by weight or less based on the weight of the hydrogel material.

The term "enzyme" describes a compound or material which is generally a protein which catalyzes a reaction between a naturally occurring molecule and another molecule which may be a naturally occurring molecule to produce a reaction product(s). An enzyme protein of the invention may be isolated from a natural source or may be recombinant.

The term "enzyme load" is used herein to refer to the amount of enzyme activity added to the gel mixture per gram of final hydrated gel. The amount of enzyme (units of activity, "units") added to the mixture is adjusted such that sufficient active enzyme is present to react quickly with the analyte such that the diffusion of analyte in the gel is the rate limiting factor. Further, sufficient enzyme is added such that manipulation of the gel in cross-linking, storage, and handling of the gel do not reduce the amount of active enzyme below the level at which analyte diffusion is the rate limiting factor.

Preferably, the enzyme load of a gel of the invention is sufficient such that the enzyme reaction is rate limiting for diffusion of the analyte in the gel. Such a condition is defined by a relationship between the gel thickness, L; the diffusion constant, D, of the analyte (such as glucose for a glucose oxidase catalyzed reaction); the enzyme load, E; the catalytic rate constant of the enzyme, $K_c$; and the Michaelis-Menten rate constant of the enzyme, $K_m$. Since diffusion-limiting enzyme reaction conditions are preferred, enzyme load and gel parameters are chosen to agree with the following relationship:

$$L(K_cE/K_mD)^{1/2} \geq 1$$

<u>Basic Structure</u>

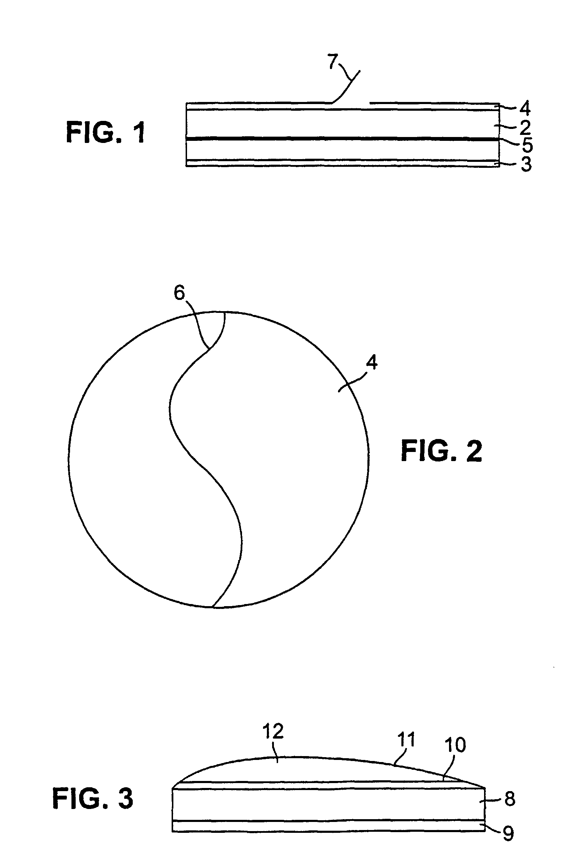

Fig. 1 is a cross-sectional schematic view of a patch such as a hydrogel patch of the invention. The basic structural patch component such as the gel patch component 2 has release liner components 3 and 4 positioned on opposite surfaces. The release liners 3 and 4 are included in order to improve the handleability of the patch in that the patch may be somewhat wet and sticky. As shown in Fig. 2 the release liner 4 may include a perforated "S-shaped" cut 6 which allows the release liner to be easily removed when the outer edges of the patch are bent towards each other. As shown in Fig. 1 an edge portion 7 of the release liner will move away from the upper surface of the patch 2 and then can be easily peeled away.

In addition to the components present within the patch component 2 which are described above when the patch component 2 is a gel it preferably includes a layer of material or fibers or a non-woven fabric 5 which is embedded within the hydrogel patch 2. The non-woven material 5 aids in improving the structural integrity of the device in that the device is comprised of a large amount of water and is particularly thin and therefore may be difficult to handle. The material layer 5 can be designed so that it provides a high degree of structural integrity to the patch without adversely effecting the flow of current through the gel.

Fig. 3 shows another embodiment of the invention. In accordance with Fig. 3 the main structural component is an absorbent material 8 which may be in the form of a sponge which can be a natural or synthetic sponge. The absorbent material is initially dry or substantially free of any water. The absorbent material 8 may be comprised of any thin layer of absorbent material and may further comprise other components such as lyophilized enzyme such as glucose oxidase. The absorbent material 8 may be bound on one surface by a release liner 9. On its other surface the absorbent material is covered by a breakable seal 10 which separates the absorbent material 8 from the contents of a package 11 which includes an aqueous solution or water 12.

When pressure is applied to the package 11 the seal 10 is broken and the aqueous contents 12 are absorbed into the absorbent material 8. The contents 12 of the package 11 are carefully measured so as to not include too much or too little water and/or its dissolved components. After the contents 12 of the package 11 are completely absorbed by the absorbent material 8 the package 11 including the breakable seal 10 are removed. The release liner 9 is also removed and the absorbent material 8 which has been saturated with water and/or solution 12 is placed in contact with the skin of the patient.

The embodiment shown in Fig. 3 is advantageous in that it can include the enzyme such as the glucose oxidase enzyme within the absorbent material in a dry state. In this state the enzyme has a longer shelf life. However, the embodiment can have certain disadvantages. For example, it is possible that all of the solution and/or water 12 in the package 11 is not completely released from the package 11 or does not absorb into the absorbent material 8 which could result in variability in terms of results obtained when using the device.

Regardless of the embodiment used all of the devices of the invention will include an enzyme which breaks down a biologically important molecule whose concentration is to be measured such as glucose and creates a measurable and predictable amount of a signal such as an electrical current based on each molecule broken down. Further, each device will include a basic structural component such as the gel 2 or absorbent material 8 through which the biologically important molecule such as glucose and any resulting reaction product may permeate. Any of the devices of the invention can also include additional components as indicated above including a buffer such as phosphate which maintains the pH within a relatively narrow range and a salt such as sodium chloride.

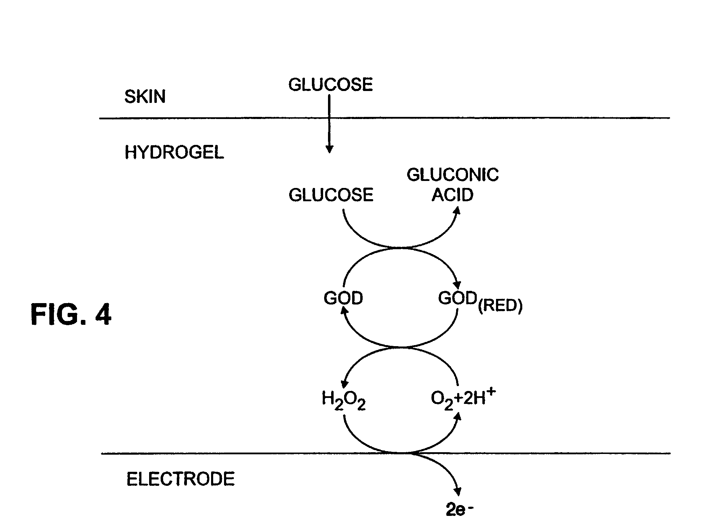

Fig. 4 is a schematic view of how the glucose oxidase (GOX) enzyme reacts with glucose entering a patch of the invention resulting in hydrogen peroxide which forms on an electrode surface provides two electrons which provide the signal in the form of electrical current which can be measured and related to the amount of glucose entering the patch.

Based on the above description of Figs. 1-4 it will be recognized that a patch of the invention can be configured in a variety of different forms from a variety of different materials. However, the patch will have certain defined mechanical, electrical, chemical and diffusion characteristics.

Description of the Embodiments

The present invention is useful in connection with the detection of biologically significant molecules such as glucose which is moved through human skin using a technique known as electroosmosis. Other techniques have been demonstrated to extract measurable quantities of glucose from body fluids such as saliva, tears, mucous, interstitial fluid, and sweat. Such techniques include, but are not limited to, sonophoresis, laser ablation, suction blisters, tape stripping, and passive diffusion with or without skin penetration enhancers.

The basic concept of moving a molecule such as a glucose through human skin is disclosed within U.S Patent 5,362,307, issued November 8, 1994 and U.S. Patent 5,279,543, issued January 18, 1994 which patents are incorporated herein by reference for disclosing the basic concept of moving molecules such as glucose through human skin by means of electroosmosis. The concept of converting the very small amounts of molecules such as glucose which can be extracted through the skin in order to create a current by use of glucose oxidase is disclosed within earlier filed European patent EP-B-0 766 578 and its divisional, European patent application EP-A-1 016 433 both of which are incorporated herein by reference in their entirety and which disclose inventions which were invented under an obligation to assign rights to the same entity as which the rights in the present invention were invented under an obligation to assign to.

A hydrogel patch or other device of the invention is placed in contact with an electrode which generates a current. The current results in moving molecules through the patient's skin and into the hydrogel patch or other device of the present invention. The glucose is broken down, as described above and shown in Fig. 4 to create hydrogen peroxide which will contact an electrode and release electrons which create an electrical current which can be detected and related to the amount of glucose entering the device.

The composition, size and thickness of the device can be varied and such variance can affect the time over which the device can be used. The hydrogel patch of Fig. 1 or device of Fig. 3 are generally designed so as to provide utility over a period of about 24 hours. After that time some deterioration in characteristics can be expected and the device should be replaced. The invention contemplates devices which are used over a shorter period of time e.g., 6 to 12 hours or a longer period of time e.g., 1 to 30 days.

In its broader sense, a patch of the invention can be used to carry out a method which comprises extracting any biomedically significant substance through the skin of a human patient and reacting that substance with another substance or substances (which reaction is greatly accelerated by the use of an enzyme e.g., 10 to 100 times or more as fast). The reaction forms a product which is detectable by electrochemical or other means by the production of a signal, which signal is generated proportionally based on the amount of a biologically important or biomedically significant substance drawn into the patch. As indicated in the above-cited patents the ability to withdraw biochemically significant substances such as glucose through skin has been established (see 5,362,307 and 5,279,543). However, the amount of compound withdrawn is often so small that it is not possible to make meaningful use of such methodology in that the withdrawn material cannot be precisely measured and related to any standard.

The present invention provides a patch which includes an enzyme which is capable of catalyzing a reaction between the biomedically significant substance such as glucose and another substance such as oxygen. In connection with the present invention the oxygen need not be added to the patch but will, infuse naturally into the patch and in the presence of glucose oxidase react with the glucose to form gluconic acid and hydrogen peroxide. The hydrogen peroxide is produced in an amount proportional to the amount of glucose drawn into the patch. The hydrogen peroxide can be detected electrochemically at an appropriate sensor by the release of two electrons producing a current proportional to the hydrogen peroxide concentration. Components of the hydrogel are chosen such that the components do not significantly degrade hydrogen peroxide and adversely affect its quantitation. Preferably, components such as catalase, polyvinyl pyrrolidone (PVP), antioxidants such as BHT and BHA, and other peroxide degradative components are reduced or limited such that quantitation of hydrogen peroxide produced by the glucose oxidase reaction is not compromised.

The invention is remarkable in that it allows for the detection and measuring of amounts of glucose which are 1,2 or even 3 orders of magnitude less than the concentration of glucose in blood. For example, glucose might be present in blood in a concentration of about 5 millimolar. However, the concentration of glucose in a patch of the invention which withdraws glucose through skin is on the order of 2 to 100 micromolar. Micromolar amounts are 3 orders of magnitude less than millimolar amounts. The ability to detect glucose in such small concentrations is attained by including the enzyme and providing a plurality of characteristics to the device including mechanical, electrical, chemical and diffusion characteristics of the type described herein. These characteristics must be carefully balanced so that the importance of one does not deteriorate the importance of another. For example, the use of radiation in order to obtain cross-linking and improve the structural integrity of the patch is important for the device to have real world commercial utility. However, radiation often deteriorates the activity of an enzyme. When producing the device it is necessary to include the enzyme prior to radiation. Thus the enzyme is radiated. However, applicants have found that by including glucose oxidase the amount of radiation sufficient to obtain the necessary degree of cross-linking does not significantly deteriorate the activity of the enzyme. The patch could be increased in thickness to improve its structural integrity, but the thickness of the patch reduces desirable diffusion characteristics and increases undesired resistance.

Description of the Functional Components of the Invention

The invention must provide some basic characteristics in order to be useful for its intended purpose which is to allow the infiltration of very small amounts of glucose from the skin of a human patient, allow the glucose to diffuse and to react in the presence of an enzyme resulting in the generation of a detectable signal such as electrons which create a current which can be measured and related to the amount of glucose entering the device. For reasons that may relate to factors such as the build up of undesired materials in the device, deterioration of the enzyme etc., the device must be easily replaceable by a patient in a convenient manner. Accordingly, the device must have some structural integrity, provide for the passage of a current and include an enzyme such as glucose oxidase.

Gel forming material: The gel of the invention includes solute material which forms network structures which hold and entrap water and thus create a gel when combined with water. However, the water may be absorbed into an absorbent material such as a thin layer of sponge or other material which absorbs a large percentage of water. The material might be hydrophilic and absorb water naturally and/or in the presence of a surfactant and/or wetting agent.

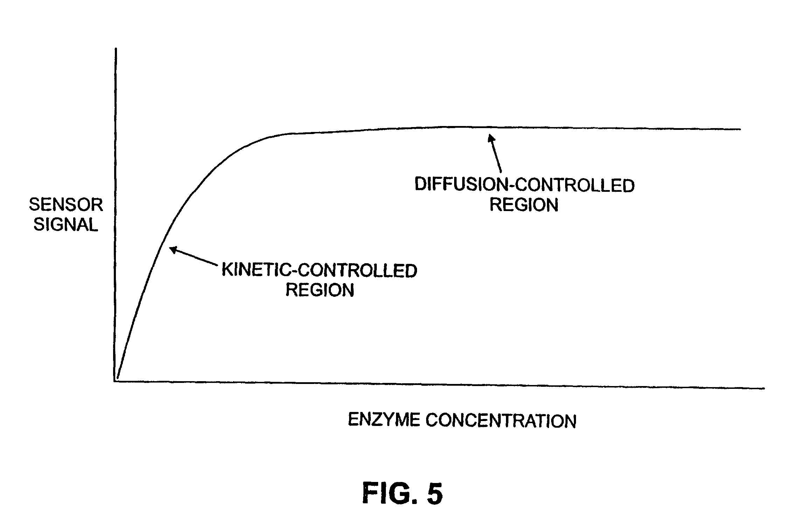

Enzyme: An essential component of the invention is an enzyme which is capable of catalyzing a reaction with a biomedically important molecule such as glucose to the extent that a product of this reaction can be sensed, e.g.,

can be detected electrochemically from the generation of a current which current is detectable and proportional to the amount of the molecule such as glucose which is reacted. A suitable enzyme is glucose oxidase which oxidizes glucose to gluconic acid and hydrogen peroxide. The subsequent detection of hydrogen peroxide on an appropriate electrode generates two electrons per hydrogen peroxide molecule which create a current which can be detected and related to the amount of glucose entering the device (see Fig. 4). Glucose oxidase (GOX) is readily available commercially and has well known catalytic characteristics. However, other enzymes could also be used provided they catalyze a reaction with a biologically significant molecule such as glucose which reaction results in the generation of a detectable product in proportion to the amount of the molecule such as glucose reacted. In that the glucose oxidase is an enzyme it can be present in relatively small amounts and the device can still be operable. This is true in that the enzyme does not enter into the reaction but merely catalyzes the reaction and therefore can be used to breakdown a large number of molecules, e.g., glucose molecules. However, in a preferred embodiment of the invention the glucose oxidase is present in sufficient amount such that any glucose entering the device is almost immediately contacted with a glucose oxidase enzyme to allow for the break down of the glucose. Stated differently the glucose oxidase is not present in such a small concentration such that large percentage amounts of glucose will be present awaiting the availability of a glucose oxidase enzyme in order to allow for the breakdown of the glucose. In general, it has been found that when a hydrogel patch of the present invention is brought into contact with human skin and current is applied to extract glucose the patch should contain a sufficient amount of glucose oxidase to allow all glucose entering to have an available enzyme molecule which is 200 units or more of glucose oxidase per gram of hydrogel. The glucose oxidase is present in an amount of from 10 units to 5,000 units per gram of hydrogel. When glucose oxidase is present at a level of 100 to 200 units or more per gram of a 5 mil thick gel, the rate of reaction of glucose at the enzyme is sufficiently high so as to react all glucose diffusing into the gel into hydrogen peroxide and gluconic acid i.e., diffusion of the analyte, glucose, is the rate limiting factor. Glucose infusing into the gel is not left unreacted while free enzyme becomes available to react with oxygen. The curve of Fig. 5 becomes substantially horizontal at a glucose oxidase concentration of about 200 units per gram of hydrogel. However, it is desirable to include excess amounts of enzyme in order to ensure that all the glucose is readily broken down into gluconic acid and hydrogen peroxide. Thus, larger amounts such as 2,000 units per gram of hydrogel should be used. This allows for the degradation of a certain percentage of enzyme when the device is stored (i.e., provide for built in shelf life), and also allow for some degradation of the enzyme during use of the device over a period of time which may be from 12 hours to one week but is more preferably about 24 hours. In order to maintain the activity of the enzyme it is useful to include enzyme stabilizing agents. The relationship between the enzyme concentration and the signal generated by a reaction with the glucose is shown in Fig. 5 and the reaction of glucose with oxygen is shown in Fig. 4.

Electrolyte: The electrolyte is another essential component of the present invention. Electrolyte must be present to allow for ionic current to flow within the water. It is preferable for the electrolyte to be a salt, such as a chloride ion. Accordingly, salts such as sodium chloride, and potassium chloride may be used in connection with the present invention with sodium chloride being particularly preferred. A buffer component of the present invention may function as a buffer as well as an electrolyte, without the addition of an additional electrolyte, such as sodium chloride. An electrolyte is added to the gel mixture such that the ionic strength of the gel is preferably between approximately 10 mM and 200 mM.

Buffer: Although it is a non-essential component a buffer is preferably used in connection with the present invention. The buffer is included in order to maintain the pH of the device within a desired range, preferably in the range of about 3 - 9. The buffer provides for useful characteristics. Firstly, the buffer maintains the pH within a range such that the glucose oxidase remains relatively stable. Secondly, the pH range is maintained near neutral so as to avoid skin irritation in that the present invention is held in contact with the skin. By stabilizing the pH the flux of glucose through the skin into the patch will not be erratic over time. A variety of useful buffers can be used in connection with the present invention. Particularly preferred buffers include phosphate buffer. However, a variety of different buffers of the type defined above with respect to the definition of the term "buffer" can be successfully used in connection with the present invention. The buffer may be various salts of phosphate, citrates, bicarbonates, succinates, acetates, and lactates.

Humectant: Another non-essential component of the invention is a humectant. The humectant is important to include in that it provides for consistency in the results obtained using the present invention. More specifically, the humectant is used in order to maintain the percentage amount of water present within the device within a very narrow range. By maintaining the water content of the gel, the device can consistently allow for the migration of the same amount of a given molecule, such as glucose, at a speed which is not erratic and allow for the flow of ions generated by the breakdown of the molecule such as glucose at the same rate. The humectant may be present in very small amounts in the range of 0.5% to 50% based on the total weight of the hydrogel patch. Useful humectants include glycerol, hexylene glycol, and sorbitol. The electrical noise contributed by the humectant is determined to be within an acceptable range for the particular gel, electrode, and operating voltage conditions contemplated. Such range is preferably less than about 200 nA, more preferably less than about 50 nA.

Cross-linker: As indicated above, the present invention is preferably provided in the form of a hydrogel which hydrogel is formed by combining polyethylene oxide with water which combination forms a gel. The structural integrity of the gel may be particularly weak when large amounts of water are present and it is desirable to include larger amounts of water in order to improve the ability of glucose and current flow through the device. However, as the amount of water increases the structural integrity of the device and its ability to be handled decreases. In order to increase the ability to handle the device and increase its structural integrity it is desirable to include a cross-linking agent. The cross-linking agent may be provided as a chemical component which provides for a reaction between different polymer chains. Alternatively, the cross-linking may be carried out by providing ionizing radiation. Such radiation is preferably provided in the form of electron beam radiation which results in linking polymer chains together. Various cross-linking agents which are used to facilitate cross-linking when used in combination with radiation are disclosed within U.S. Patents 4,684,558 and 4,989,607 both of which are incorporated herein by reference to disclose cross-linking agents and methods of radiation used in connection with the formation of gels. Useful cross-linking agents for use with U.V. radiation include N,N'-methylenebisacrylamide, polypropylene glycol monomethacrylate; polypropylene glycol monoacrylate; polyethylene glycol (600) dimethacrylate; Triallylisocyanurate (TAIC); Diallylisocyanurate (DAIC); polyethylene glycol (400) diacrylate; SR 415 Ethoxylated Trimethylolpropane Triacrylate; SR 9035 Ethoxylated Trimetholpropane Triacrylate. For cross-linking using U.V. radiation, a photoinitiator may be used. Examples of such photoinitiators include: Esacure® KB1 Benzyldimethyl Ketal; Esacure® TZT Trimethylbenzophenone Blend; Esacure® ITX Isopropylthioxanthone; Esacure® EDB Ethyl 4-(dimethylamino) Benzoate; BP Benzophenone.

E-beam radiation and gamma radiation cross-linking agents useful in the invention include, but are not limited to, ethylene glycol methacrylate, triethylene glycol methacrylate, trimethylolpropane trimethacrylate (Sartomer® 350, Sartomer Company, Exton PA, USA), and N,N'-methylenebisacrylamide.

Thermal and chemical cross-linking agents useful in the invention include, but are not limited to, ethylene glycol methacrylate, triethylene glycol methacrylate, trimethylolpropane trimethacrylate (Sartomer® 350), N,N'-methylenebisacrylamide, and glutaraldehyde. Useful initiators of cross-linking include, but are not limited to, azobisisobutyronitrile (AIBN), and benzoyl peroxide.

Cross-linking agents are added to the gel mixture in an amount that allows the desired physical properties of the gel as described above. The amount of residual cross-linking agent present in the gel following cross-linking is preferably in an amount that is not toxic to the patient when the gel is contacted with the patient's skin for the time the gel patch is in use.

Biocide: As indicated above, the hydrogel patch or other device of the invention is intended to be used in contact with human skin. Further, the device may be packaged and stored for relatively long periods of time prior to use. In view of such it may be desirable to incorporate a biocide compound within the device. Such biocide is present in an amount sufficient to kill and/or inhibit the growth microorganisms of the type described above in the definition of "biocide".

The physical characteristics of the gel:

Diffusion: With respect to diffusion characteristics, the patch must be capable of allowing for the infusion of a biologically significant molecule such as glucose from the skin and the movement of the molecule and its reaction products (e.g., gluconic acid and hydrogen peroxide) through the patch to the extent necessary to ultimately result in the generation of a detectable signal such as electrical current. The hydrogel patch as per Example 5 allows for the diffusion of hydrogen peroxide at $8 \times 10^{-6}$ $cm^2$/sec and glucose at $1 \times 10^{-6} cm^2$/sec. For example, rates greater than about $10^{-6}$ $cm^2$/sec and $10^{-7}$ $cm^2$/sec for hydrogen peroxide and glucose, respectively, are preferred. It will be understood that diffusion characteristics are related, to some extent, to mechanical characteristics and that all of the characteristics of the device are interrelated to each other in order to obtain a desired end result which is a disposable device which proportionally converts a molecule such as glucose entering the device to a predetermined amount of signal such as electrical current which can be measured. In preferred embodiments of the invention, the patch had a resistance of not more than approximately 20 Kohms and preferably not more than approximately 1 Kohm after contact with the skin for a 24 hour period.

The characteristic time of the gel is measured as described above as a function of the thickness of the gel (L, the distance the analyte diffuses) and the diffusion constant of the analyte (D). The relationship between the parameters L and D is the following:

$$L^2/D = \text{Characteristic time, minutes}$$

Preferably, the characteristic time of a gel of the invention is approximately 6 seconds to 45 minutes. Preferably, measurement of analyte in the gel occurs continually (e.g., measurements may be integrated over 5 minutes and occur every 20 minutes over a day). Preferably D for a particular analyte in the gel should be no slower than 0.1 times the diffusion rate of the analyte in water alone. More preferably, D for a particular analyte in the gel is more than 0.25 times the diffusion rate in water. Cross-linking of the gel may be varied to make diffusion of the analyte the rate limiting factor in detection.

Gel cohesion: The hydrogel patch form of the invention and other forms, are preferably slightly tacky and will adhere to human skin and conform to the configuration of the skin over which the patch is applied. Thus the patch will be flexible and tacky to the extent that it will adhere to skin and not fall off due to gravity. Further, when removed the patch will not be sufficiently adhesive such as to tear away skin and can be removed and will not adhere to the skin on being removed so as to leave a tactile hydrogel residue on the skin following removal.

Electrical Conductivity: Electrically the patch must provide for sufficient electrical conductivity and should have a resistance of no more than approximately 20 Kohms, and preferably no more than approximately 1 Kohms after being in contact with the skin for a 24 hour period. Further, the patch preferably creates an electrical environment such that background noise created when the patch is used is as close to zero as possible. Preferably, the amount of background noise is less than 500 nA, more preferably less than 200 nA, and most preferably less than 50 nA when measured on a cross-linked gel.

Structural Support: The hydrogel patch may further include a structural support which is embedded in the gel, which support includes, but is not limited to, a woven fabric, a non-woven fabric, dispersed fibers, or a membrane. In addition it is possible to include a membrane which aids in filtering out undesirable materials which are drawn into the hydrogel patch. This structural support is embedded in the gel and preferably has a size and configuration which matches that of the hydrogel patch. A variety of different materials can be used to provide the structural support. Useful non-woven fabrics include those sold as Reemay 2200, 2000 and 2400 series. The layer may be spunbonded polyester which may be straight or crimped fibers. It is possible to use super absorbent fibers or fabrics. Commercially available materials include Camelot Fiberdre®, Verlée (non-woven), Dupont Sontara® (polyester blend fabrics) and Kendall non-woven fabrics. Open-cell and closed-cell materials can be used.

Chemical Characteristics: The chemical characteristics of the patch must provide an environment such that degradation or deterioration of the substance being measured (such as hydrogen peroxide) is no more than 20% over a period of about 30 min. Further, an environment is provided such that the enzyme is not significantly deteriorated and the skin is not significantly irritated. Preferably, a sufficient amount of enzyme is present in the hydrogel such that diffusion of the analyte through the gel is the rate limiting factor in analyte measurement. Thus, the patch is preferably maintained within a pH range of from about 3 to 9. Preferably the pH is adjusted to allow an optimal rate of conversion of $\alpha$-glucose to $\beta$-glucose since glucose oxidase converts $\beta$-glucose to gluconic acid at a rate 150 times greater than the rate of $\alpha$-glucose. The term optimal refers to a balance of several parameters within the gel, including, but not limited to, enzyme stability, ionophoretic flux of glucose, skin irritation, and the like. The ratio of $\beta$-glucose:$\alpha$-glucose is approximately 2:1. A pH of approximately equal to or greater than 7 or equal to or less than 4 is preferred to enhance the rate of mutarotation. Conditions under which total glucose ($\alpha$-glucose and $\beta$-glucose) is converted to peroxide is less than the measurement time ($t_m$), preferably less than one-third of the measurement time. Such conditions include, but are not limited to a phosphate buffer concentration greater than or equal to about 10 mM, a pH greater than or equal to about pH 7 or less than or equal to about pH 4, or the addition of the enzyme, mutarotase. However, to some extent chemical and electrical characteristics are interrelated. Thus, in addition to maintaining to maintaining the pH of the gel at a level to promote enzyme stability and $\alpha$-glucose to $\beta$-glucose mutarotation, the pH is chosen to enhance iontophoretic flux. These parameters are further balanced to minimize the skin irritation of the user.

The hydrogel of the invention is provided in two principal aspects: a gel patch which is pre-hydrated prior to manipulation by the patient, and a gel patch which is dry and is hydrated by the patient just prior to use. The features of the final hydrated gel is as described above for each aspect of the invention. General features of the pre-hydrated gel and the dry gel are provided below.

Hydrated Gel: In order to achieve the objects of the invention the device can be constructed in a number of different configurations. The basic concept is to provide a component which allows for a large percentage of water to be present and held in place through which various molecules (e.g., ions) may readily diffuse and into which glucose may be infused. The presently preferred configuration is to use a hydrogel patch which is comprised of a gel forming material which forms one or more structures such as a network which holds water and forms a gel in the presence of water.

The gel forming material is present as a single component or multiple gel forming components, the sum of which is present in an amount from about 0.5% to about 40% by weight based on the total weight of the hydrogel patch. In a particularly preferred embodiment of the invention, polyethylene oxide is present in an amount of about 2% to 20%, more preferably about 10%. If polyacrylic acid is present, it is added in an amount in the range of about 0.5% to 5%, more preferably 2%. Water is present in an amount of 45 - 95% or preferably about 65 - 80% which water includes other components in solution.

Apart from the gel forming material, the remainder of the patch is comprised of a water solution wherein the water necessarily includes an enzyme. Where the desired measurement is the detection of glucose, the enzyme is preferably glucose oxidase. An amount of enzyme is added such that the enzyme in the final gel used by the patient is sufficiently active so that analyte diffusion through the gel remains the rate limiting factor for the measurement. The amount of enzyme (enzyme load) will vary with the enzyme and gel manipulation processes. Processes which can potentially degrade the enzyme include, but are not limited to, gel pH, cross-linking conditions, storage temperature, light, pH change, and use by the patient. Thus the enzyme load will compensate for the potential loss of enzyme activity due to these procedures. For example, where glucose oxidase is the enzyme, approximately at least 1000 units, preferably 2000 units per gram of gel are used. It is within the scope of the invention that the enzyme load may be varied (increased or decreased) as gel manipulation processes are varied. Finally, the enzyme added to the gel may be from natural sources, such as by isolation from an organism, or the enzyme may be produced by recombinant or chemical means.

Another component of the gel is a salt which renders the water electrically conductive. Such a salt is preferably sodium chloride. The solution may include other components such as a buffer which maintains the pH of the hydrogel patch in the range of about 3 - 9. The chloride salt may be excluded from the gel where a buffer salt is included in the gel and provides sufficient electrical conductivity while also maintaining an optimal pH.

The gel components may further include, but are not limited to, a biocide (such as methylparabens), a humectant (such as sorbitol, hexylene glycol, or glycerol), and an ionic or non-ionic surfactant (such as poloxamer). The gel may further include a cross-linking agent which, with radiation, thermal activation, or chemical activation, enhances cross-linking thereby providing greater structural integrity.

It is desirable to provide a basic material which includes as large amount of water as possible in that a greater amount of water provides a device which more easily allows for the infusion of glucose and the conduction of current therein. However, as the amount of water increases the ability to easily handle the device and allow the device to maintain its components and structural integrity is decreased. For this reason it is often desirable to use a gel which is comprised of a synthetic polymeric materials such as polyvinyl pyrrolidone or polyethylene oxide (such as Polyox® WSR-NF grade) in combination with polyacrylic acid (such as Carbopol®), which polymers may be cross-linked by using a chemical cross-linking agent or by the application of radiation such as can be provided by electron beam radiation or U.V. radiation.

A variety of different types of gel forming materials are known to those skilled in the art. For example, materials for forming hydrogel are disclosed within U.S. Patent 4,684,558 and highly conductive adhesive hydrogels are disclosed within U.S. Patent 4,989,607 both of which are incorporated herein by reference to disclose and describe materials used in the formation of hydrogels, methods of forming such hydrogels and various materials and devices which can be used in connection with the formation of such hydrogels. These patents each cite numerous other U.S. patents and other publications which disclose other materials which are used in the formation of gels and those publications are also incorporated herein by reference. Lastly, it is pointed out that it is possible to use a gel such as that disclosed within PCT Publication WO93/10163, published May 27, 1993 which discloses gels which can be used in the formation of patches for the long term application of pharmaceutically active agents to a patient which publication is incorporated herein by reference to disclose such gels.

Dry Gel: In yet another aspect of the invention a solute material, such as an absorbent material, is provided which material may be in the form of a sponge which can be natural or synthetic or a fibrous paper, polyethylene oxide, Carbopol®, Loprasorb®, polyester, polyester mesh, and other like material which is hydrophilic. This thin layer of absorbent material may have essential components, in a dry state, embedded therein. For example, the material may include lyophilized glucose oxidase and sodium chloride as well as a pH buffer such as phosphate or bicarbonate. In one embodiment, this solute material with the dried components embedded therein is provided along with a predetermined amount of water or solution in a breakable package. When the patient applies pressure to the package the water is released to the absorbent material which absorbs the water and brings the enzyme salt and buffer in the solution within the absorbent material. The water may contain other components such as a biocide or humectant. In alternative embodiments the solution may include the salt, enzyme and buffer. However, it is more preferable to include, at least, the enzyme within the absorbent material in a dry lyophilized state in that the enzyme is more stable in a dry state than when contained within solution.

In another embodiment of the invention, the absorbent material with the dried components embedded therein is provided such that the patient merely adds water or saline in order to hydrate the material and form the gel.

One preferred hydrated gel includes an amount of greater than 4% and preferably less than 35% by weight of cross-linked polyethylene oxide having a weight average molecular weight of from about 0.02-6 x $10^6$ daltons which material is subjected to high energy radiation from about 0.2 to about 5.0 Mrads. Specific physical characteristics and tests used in measuring those characteristics are disclosed within U.S. Patent 4,684,558. In addition to using polyethylene oxide it is possible to use various mixtures of polyethylene oxide alone or in combination with another polymer forming materials. In preferred embodiments, the polymer forming materials do not adversely affect quantitation of the analyte. Polyethylene oxide can be used by itself or in combination with viscosity-enhancing hydrophilic polymers as disclosed within U.S. Patent 4,989,607.

EXAMPLES

The following examples are put forth so as to provide those of ordinary skill in the art with a complete disclosure and description of how to make patches of the present invention. Efforts have been made to ensure accuracy with respect to numbers used, (e.g., amounts, particular components, etc.) but some experimental errors and deviations should be accounted for. Unless indicated otherwise, parts are parts by weight based on the total weight of the hydrogel, components dissolved in water are measured as a percentage of the solution, molecular weight is weight average molecular weight, temperature is in degree centigrade, and pressure is at or near atmospheric.

EXAMPLE 1

This example describes non-limiting methods for characterizing some of the physical properties of gels of the invention. Gels described in Table 1, below, were prepared as described herein and tested by the procedures given below.

TABLE 1

| Component | Formulation Numbers | | | | |
|---|---|---|---|---|---|
| | 60 | 63 | 70 | 101 | 103 |
| Polyox® WSR 205, % | 7.5 | 7.5 | 7.5 | 7.5 | 8.5 |
| Carbopol® 910 P NF, % | | | | | 2 |
| Carbopol® 974 P NF, % | | | 1 | 2 | |
| KC1, % | 5 | | | | |
| NaCl, % | | .45 | .45 | .45 | .45 |
| NaHCO$_3$, % | | .5 | .5 | .5 | .5 |
| Glycerol, % | 10 | 10 | 10 | | 10 |
| Hexylene glycol, % | | | | 10 | |
| Bisacrylamide, % | 2 | 2 | 2 | .5 | .5 |
| Water, nanopure, % | 75.5 | 79.55 | 78.55 | 79.05 | 78.05 |

Formulation numbers refer to the 316 series. Weights of components are percentages based on the weight of the hydrated gel. Each formulation contained 100 Units of glucose oxidase per gram of gel. Bisacrylamide refers to N,N'-methylenebisacrylamide.

The components of the gel mixture described above were adjusted such that the physical characteristics of the final gel was optimized for quantitation of an analyte, such as glucose, drawn through the skin of a patient, reacted, and its reaction product detected and quantitated. In that relatively small amounts of glucose enter the device, it is necessary that the device be particularly thin e.g., in the range of 5 $\mu$m to 1.27 mm (50 mils) (1 mil equals one one thousandth of an inch), preferably 0.025 to 0.25 mm (1 to 10 mils). Its overall surface area on a single surface should be in the range of about 0.5 cm$^2$ to about 10 cm$^2$ and is more preferably in the range of about 1 to about 5 cm$^2$.

Cohesiveness of the gel is another characteristic that may be optimized. A gel of the invention, once hydrated, has sufficient structural integrity so as to maintain its shape within the device, conforms to the contours of the patient's skin when applied thereto, and does not adhere to the patient's skin to such a degree that portions of gel material are torn away and left on the patient's skin when the gel is removed.

Cohesiveness of the gel monitored by measuring tack using a rolling ball tack test as follows. A steel ball of approximately 16.5 mm diameter was rolled down a gel-free inclined plane. The steel ball was next rolled down a similarly inclined plane upon which a 2.5 cm x 30.5 cm (1 inch x 12 inch) strip of the hydrogel was adhered. The distance traveled by the steel ball on each of the surfaces was measured and compared. Increased cohesiveness (tack) of the gel is observed as a shortening of the distance traveled. In preferred embodiments of the gel, the cohesiveness, measured as tack, is less than approximately 30 mm. For example, formulations 316-101 and 316-103 from Table 1 had tack values of 28.4 mm $\pm$ 8.0 mm and 19.2 mm $\pm$ 6.9 mm, respectively.

Electrical quietness is another characteristic of the gel of the invention which refers to the low level of background electrical noise that is achievable according to the invention, which low level of noise improves the capability of the invention to detect small quantities of analyte. Preferably, the patch creates an electrical environment such that background noise created when the patch is used is as close to zero as possible. Preferably, the amount of background noise is less than 500 nA, more preferably less than 200 nA, and most preferably less than 50 nA when measured on a cross-linked hydrogel.

Background current (noise) is measured by the following procedure. A rectangular electrode assembly consisting of a working and a counter Pt electrode and a reference Ag/AgCl electrode was used. A 1.59 cm (5/8 inch) diameter hydrogel disk was cut out, one release liner removed, and the disk was placed on a rectangular electrode with the adhesive side toward the electrode. The background current was measured for an applied potential of 0.6V. The electrode was preconditioned at a bias potential of 0.75V for 10 min before starting the background current measurement. The background current measurement decays asymptotically to a steady background current within approximately 15 to 30 minutes. Measurement was taken at approximately 60 min. Preferably, the background current is less than approximately 500 nA, more preferably less than approximately 200 nA, and most preferably less than approximately 50 nA.

In a preferred embodiment of the invention, the gel components were treated to remove compounds that cause a relatively high background electrical signal. For example, additives in the gel components such as the antioxidants present in commercial polymers are electroactive. Such electroactive compounds may be removed by a clean up procedure such as, but is not limited to, diafiltration on the polymer forming materials. For example, the gel prepared in Example 2 below had a background current of 175 nA before polymer clean up by diafiltration, and a background current of 40 nA after clean up by diafiltration. Background currents were measured at 60 min following application of the 0.6V potential.

Electrical resistivity was measured by the following procedure. Two Ag/AgCl hook-shaped electrodes printed on a ceramic plate were used. A 1.59 cm (5/8 inch) diameter hydrogel disk was cut out and both release liners were removed. The hydrogel disk was placed between the ceramic plates such that the electrodes were completely covered by the hydrogel. A constant current of 0.9 mA was applied across the gel using a protocol in which the polarity was alternating with a cycle time of 15 min and the voltage drop across the gel was measured. The resistance was then calculated. In preferred embodiments of the invention, the resistance was not more than approximately 20 Kohms. Prior to contact with the skin, gels 316-60, 316-63, and 316-70 of Table 1 were tested for resistance and found to exhibit resistances of 2.7, 3.9, and 2.2 Kohms, respectively. Preferably the resistance is not more than approximately 20 Kohm after contact with the skin for a 24 hour period.

EXAMPLE 2

Polyethylene oxide (PEO, Polyox® WSR-205) (approximately 8.5% by weight) was combined with polyacrylic acid PAA (Carbopol® 971 P NF) (2% by weight), hexylene glycol (10% by weight), N,N'-methylenebisacrylamide (0.02% by weight), poloxymer 188 (Pluronic® F68) (0.5% by weight) and approximately 75.5% water solution wherein the water contained 200 units of glucose oxidase per gram of gel, 0.45% NaCl and sufficient phosphate buffer to maintain the pH in the range of 6 - 8. The weights of PEO, PAA and water solution are based on the total weight of the hydrogel produced and the percent amounts of the NaCl and buffer are percent amounts of these components in the gel.

The components were mixed at ambient temperature, and the electrical characteristics of the gel measured.

Cross-linking was performed as follows: The gel mixture was cross-linked by first coating the gel mixture onto a support substrate and subjected to about 0.35 to 0.45 Mrad irradiation at ambient temperature.

Water loss of the gel was measured as follows: The gel, 1 mm (40 mils) thick, approximately 1.9 cm (0.75 inches) in diameter, was placed between circular disks of release liners such that water vapor could escape only from the sides of the gel. Weight loss was measured at selected time points over a period of 24 hours at ambient temperature and pressure. Weight loss was attributed to water loss, and was normalized to the initial water content of the gel. A water loss from the gel of less than 70% over 24 hours was observed.

A list of components of an example hydrogel of the invention is provided in Table 2.

TABLE 2

| A Hydrogel Formulation | |
| --- | --- |
| Polyox® WSR-NF | 8.5% |
| Carbopol® 971 P NF | 2% |
| Hexylene glycol | 10% |
| NaCl | 0.45% |

TABLE 2   (continued)

| A Hydrogel Formulation | |
|---|---|
| Phosphate buffer | 0.5% |
| Pluronic® F68 | 0.5% |
| N,N'-methylenebisacrylamide | 0.02% |
| Glucose oxidase | 0.16% (200 U/g gel) |
| Water | 75.5% |

EXAMPLE 3

A high polymer content gel was prepared by combining the following components: polyethylene oxide (PEO, Polyox® WSR-750) (approximately 20% by weight), N,N'-methylenebisacrylamide (0.02% by weight) and approximately 78.05% water solution wherein the water contained 1000 Units of glucose oxidase per gram of gel, 0.45% NaCl and 0.5% sodium bicarbonate. The weights of PEO and water solution are based on the total weight of the hydrogel produced and the percent amounts of the NaCl and buffer are percent amounts of these components in the gel. The components were mixed gently at ambient temperature.

Cross-linking was performed as follows: The gel mixture was cross-linked by first coating the gel mixture onto a support substrate and subjected to about 0.35 to 0.45 Mrad irradiation at ambient temperature.

EXAMPLE 4

Provide a synthetic sponge material having a thickness of 0.64 mm(25 mils) and a diameter of 1 cm. The sponge material is incorporated with lyophilized glucose oxidase enzyme in an amount of 1,000 units per gram of gram of sponge material present in an attached package which package incorporates approximately 3 milliliters of water separated from the sponge by a breakable seal which seal is broken upon the application of pressure to the package which pressure breaks the seal but not the remainder of the package. The water in the package has dissolved therein 0.5% sodium chloride and phosphate buffer sufficient to provide a pH of about 6 - 8.

EXAMPLE 5

Combine 5.5% by weight of polyethylene oxide (PEO 750 having a molecular weight of about 300,000), 1% by weight of polyacrylic acid PAA (Carbopol® 974 P NF) and approximately 91.75% water solution wherein the water contains 1,000 units of glucose oxidase per gram of gel, 0.45% NaCl and phosphate buffer to maintain the pH in the range of 6 - 8. The weights of PEO, PAA and water solution are based on the total weight of the hydrogel produced and the percent amounts of the NaCl and phosphate buffer are percent amounts of these components in the water solution. The gel incorporates a polyester non-woven material sold as Reemay 2250. In order to produce the patch the mixtures of components are gel cast on the non-woven material which is on a release liner layer. The gel is cast with a Gardner knife and laminated to a second release liner layer. The material is subjected to E-beam radiation in an amount of about 0.4 Mrad to cross-link. The material is die cut to a circle having a diameter in the range of 1 to 3 cm and will have a thickness in the range of 0.25 to 1 mm (10 to 40 mils). The circular disc is placed in a sealed pouch to prevent evaporation or contamination.

EXAMPLE 6

A polyethylene oxide/polyvinyl alcohol gel was prepared as follows. The following components were combined per 100 grams of gel: 8.5 g of polyethylene oxide (PEO, Polyox® WSR 205), 10 g of polyvinyl alcohol (Airvol® 203S), 2 g of polyacrylic acid PAA (Carbopol® 971 P NF), 2 g N,N'-methylenebisacrylamide, and approximately 74.6 g water solution wherein the water contained approximately 100 Units per gram of gel of glucose oxidase, 0.45 g of NaCl, and 0.26 g $Na_2H_2PO_4.H_2O$ and 2.17 g of $Na_2HPO_4.7H_2O$ phosphate buffer and the pH was maintained at pH 7.4. Radiation in the form of E-beam radiation was carried out to induce cross-linking. The weights of all components are based on 100 grams total weight of the hydrogel produced. The weight of glucose oxidase is per gram of gel. The components from a gel which can be formed into a patch having a circular parameter with an area of about 1 $cm^2$ and a thickness of about 0.13 mm (5 mils). A release liner was applied to each surface of the gel patch, which release liner has the same area and outer parameter configurations as the gel patch.

EXAMPLE 7

The following hydrogel components were combined: 8.5% by weight of polyethylene oxide (PEO, Polyox® WSR-205) having a molecular weight of about 600,000), 2% by weight of polyacrylic acid PAA (Carbopol® 971 P NF) and approximately 89.5% water solution wherein the water contains 1,000 Units of glucose oxidase per gram of gel, 0.45% NaCl and phosphate buffer sufficient to maintain the pH in the range of 6 - 8. The weights of PEO, PAA and water solution are based on the total weight of the hydrogel produced and the percent amounts of the NaCl and buffer are percent amounts of these components in the water solution. The gel incorporates a polyester non-woven material such as Reemay 2250. In order to produce the patch, the mixture of components was combined with a U.V. photosensitizer (e.g., 0.5% Irgacure® 184) and a cross-linker (e.g., 0.02% N,N'-methylenebisacrylamide), and gel cast on the non-woven material which is on a release liner layer. The gel is cast with a Gardner knife and laminated to a second release liner layer. The material is subjected to U.V. radiation to obtain cross-linking. The material is die cut to a circle having a diameter in the range of 1 to 3 cm and will have a thickness in the range of 0.25 to 1 mm (10 to 40 mils). The circular disc is placed in a sealed pouch to prevent evaporation or contamination.

Example 8

A dry gel of the invention is prepared by first preparing a hydrated gel on a solid support followed by drying of the gel on that support. The gel is rehydrated by the patient by the addition of water or saline.

Combine 10% by weight of polyethylene oxide (Polyox® WSR-750) having a molecular weight of about 300,000, 1% by weight of polyacrylic acid PAA (Carbopol® 974 P NF) and approximately 89% water solution wherein the water contains 2,000 units of glucose oxidase per gram of gel, 0.45% NaCl and 0.5% phosphate buffer to maintain the pH in the range of 6 - 8. The weights of PEO, PAA and water solution are based on the total weight of the hydrogel produced and the percent amounts of the NaCl and buffer are percent amounts of these components in the water solution. The gel incorporates a polyester non-woven material such as Reemay 2250. In order to produce the patch the mixtures of components are gel cast on the non-woven material which is on a release liner layer. The gel is cast with a Gardner knife and laminated to a second release liner layer. The material is subjected to E-beam radiation in an amount of about 0.4 Mrad to cross-link. The material is die cut to a circle having a diameter in the range of 1 to 3 cm and will have a thickness in the range of 0.25 to 1 mm (10 to 40 mils).

To prepare the dry gel, the circular disc is placed on a solid support and dried in a lyophilizer or other drying apparatus such that substantially all unbound water is removed. In addition, the conditions are chosen such that upon rehydration, the enzyme in the gel has sufficient activity to withstand storage and use, and that analyte diffusion is the rate limiting factor in the measurement of analyte.

**Claims**

1. A hydrogel patch comprising;

   (a) a hydrophilic compound which forms a hydrogel in the presence of water, which compound is present in an amount of 4% or more by weight based on the weight of the hydrogel;
   (b) water in an amount of 95% or less based on the weight of the hydrogel;
   (c) an electrolyte, wherein background electrical signal in the gel is less than approximately 200 nA; and
   (d) glucose oxidase present in an amount of from 10 units to 5,000 units per gram of the weight of the hydrogel, wherein (i) the glucose oxidase can catalyse a reaction between glucose and oxygen resulting in the generation of hydrogen peroxide, and (ii) hydrogen peroxide degradative components of the enzyme composition are reduced such that quantitation of hydrogen peroxide produced by the glucose oxidase reaction is not compromised;
   wherein the hydrogel is maintained within a pH range of from 3 to 9.

2. The hydrogel patch of claim 1, wherein said background electrical signal in the gel is less than approximately 50 nA.

3. The hydrogel patch of any preceding claim, wherein a product of the reaction in step (d) is not degraded more than 20% in 30 minutes.

4. The hydrogel patch of any preceding claim, wherein diffusion of an analyte that reacts in the reaction of step (d) is rate limiting, and wherein diffusion of the analyte is more rapid than the measurement time.

5. The hydrogel patch of any preceding claim, wherein the hydrogel has the property defined by the formula:

$$L(K_c E/K_m D)^{1/2} \geq 1$$

wherein L is the hydrogel thickness, D is the diffusion constant of an analyte drawn into the hydrogel, E is the enzyme load of the hydrogel, $K_c$ is the catalytic rate constant of the enzyme, and $K_m$ is the Michaelis-Menten rate constant of the enzyme.

6. The hydrogel patch of any preceding claim, wherein the hydrogel further comprises components for maintaining a selected hydrogel environment, and wherein the environment enhances the conversion of analyte to product of the reaction in step (d).

7. The hydrogel patch of any preceding claim, wherein the enzyme catalyses a reaction between glucose and oxygen resulting in the generation of electrons.

8. The hydrogel patch of any preceding claim, wherein the enzyme is a recombinant or synthetic glucose oxidase.

9. The hydrogel patch of any preceding claim, wherein the enzyme is present in an amount of 200 units or more of enzyme per gram weight of the hydrogel.

10. The hydrogel patch of any preceding claim, further comprising a mutarotase enzyme.

11. The hydrogel patch of any preceding claim, further comprising:

   (e) a buffering agent present in an amount sufficient to maintain a pH in the hydrogel in a range of from 3 to 9.

12. The hydrogel patch of claim 11, wherein the buffering agent is present in an amount sufficient to maintain the pH of the hydrogel in a range of from 6 to 8.

13. The hydrogel patch of claim 11 or 12, wherein the buffering agent is present in an amount sufficient to maintain the pH of the hydrogel at 7.4.

14. The hydrogel patch of claim 11, 12 or 13, wherein the buffering agent comprises a phosphate buffer.

15. The hydrogel patch of any preceding claim, wherein the hydrophilic compound is selected from one or more of polyethylene oxide, polyvinyl alcohol, polyacrylic acid and polyacrylamidomethylpropane-sulfonate and copolymers thereof.

16. The hydrogel patch of any preceding claim, wherein the hydrophilic compound further comprises bisacrylamide.

17. The hydrogel patch of any preceding claim, wherein the electrolyte is a chloride salt.

18. The hydrogel patch of any preceding claim, further comprising a biocide.

19. The hydrogel patch of claim 18, wherein the biocide is an antibacterial agent.

20. The hydrogel patch of claim 18, wherein the biocide is an antifungal agent.

21. The hydrogel patch of claim 18, wherein the biocide is selected from chlorinated hydrocarbons, organometallics, hydrogen releasing compounds, metallic salts, organic sulphur compounds, quaternary ammonium compounds, phenolics and methylparabens.

22. The hydrogel patch of claim 21, wherein the biocide agent is a methylparaben.

23. The hydrogel patch of any preceding claim, further comprising a humectant.

24. The hydrogel patch of claim 23, wherein the hydrophilic compound is present in an amount in the range of from 8% to 12% based on the weight of the hydrogel containing the humectant.

25. The hydrogel patch of any preceding claim, wherein the hydrophilic compound is selected from one or more of polyethylene oxide, polyvinyl alcohol, polyacrylic acid and polyacrylamidomethylpropanesulfonate and copolymers thereof,

wherein the electrolyte is NaCl or KCl,

wherein the enzyme is glucose oxidase and glucose oxidase is present in an amount in a range of 10 units to 5,000 units per gram of the sum of component (a) and component (b), and

which further comprises a buffering agent dissolved in the water, which buffering agent is present in an amount sufficient to maintain the pH of the hydrogel in the range of from 3 to 9.

26. A hydrogel patch consisting essentially of:

(a) a hydrophilic compound, which forms a gel, present in an amount in the range of from 0.5% to 40% based on the weight of the hydrogel when a humectant is also added or present in an amount of from 15% to 20% based on the weight of the hydrogel when no humectant is added;
(b) water in an amount of 95% or less based on the weight of the hydrogel;
(c) an electrolyte, wherein background electrical signal in the gel is less than approximately 200 nA; and
(d) glucose oxidase present in an amount of from 10 units to 5,000 units per gram of the weight of the hydrogel, wherein (i) the glucose oxidase can catalyse a reaction between glucose and oxygen resulting in the generation of hydrogen peroxide, and (ii) hydrogen peroxide degradative components of the enzyme composition are reduced such that quantitation of hydrogen peroxide produced by the glucose oxidase reaction is not compromised.

27. The hydrogel patch of claim 1, wherein (a) the hydrophilic compound comprises polyethylene oxide, (b) the water comprises a buffering agent and the buffering agent is a phosphate buffer, and (c) the electrolyte comprises sodium chloride.

28. The hydrogel patch of any preceding claim, wherein the hydrophilic compound is present in an amount of less than 40% by weight and the water is present in an amount of more than 60% by weight based on the weight of the hydrogel.

29. The hydrogel patch of any preceding claim, **characterised by** a flat configuration having a thickness in a range of 5 µm to 1.52 mm (60 mils).

30. The hydrogel patch of claim 29, **characterised by** a first and a second surface area,
wherein each surface area is in a range of 0.5 cm$^2$ to 10 cm$^2$ and wherein the hydrogel patch has a thickness of from 5 µm to 0.25 mm (10 mils).

31. The hydrogel patch of any one of claims 1 to 28, wherein said hydrogel patch has a thickness of about 0.13 mm (5 mils) and a surface area of about 1 cm$^2$.

32. The hydrogel patch of any one of claims 1 to 28, wherein said hydrogel patch is a circular disc having a thickness of 0.25 mm (10 mils) to 1 mm (40 mils) and a diameter of 1 cm to 3 cm.

33. The hydrogel patch of claim 32, wherein said hydrogel patch has a thickness of 1 mm (40 mils) and a diameter of 1.9 cm (0.75 inches).

34. The hydrogel patch of claim 32, wherein said hydrogel patch has a thickness of 0.64 mm (25 mils) and a diameter of 1 cm.

35. The hydrogel patch of any preceding claim, further comprising a structural support material embedded in the hydrogel patch.

36. The hydrogel patch of claim 35, wherein the structural support material is a non-woven fabric having an outer parameter configuration and size substantially equal to that of the hydrogel patch.

37. The hydrogel patch of any preceding claim, wherein the hydrogel patch has first and second surfaces, said hydrogel patch further comprising first and second release liners respectively disposed on the first surface and the second surfaces, and a non-woven material embedded in the material which holds water in place.

38. The hydrogel patch of any preceding claim, wherein said hydrogel patch is in the form of a thin, flat disc which will conform to the contours of the human skin and may optionally have a non-woven fabric or porous membrane embedded therein.

39. The hydrogel patch of any preceding claim, wherein one or more components of the gel have been treated to remove compounds that cause background electrical signal.

40. The hydrogel patch of claim 39, wherein one or more of said gel components have been treated using a diafiltration procedure to remove electroactive compounds therefrom.

41. The use of the hydrogel patch of any one of claims 1 to 40 for the manufacture of a device for use in a diagnostic method for electroosmotically extracting glucose through the skin of a human and into said hydrogel patch, wherein said diagnostic method comprises the steps of:

(i) applying the device comprising the hydrogel patch to the skin of the human, said hydrogel patch being in contact with an electrode, and
(ii) generating an electrical current that moves the glucose through the skin and into the hydrogel patch.

42. The use of the hydrogel patch of any one of claims 1 to 40 for the manufacture of a device for use in a diagnostic method for detecting an amount of glucose in a human,
wherein said diagnostic method comprises the steps of:

(i) extracting glucose through the skin of the human using the device comprising the hydrogel patch in contact with an electrode,
(ii) generating an electrical current that moves the glucose through the skin and into the hydrogel patch, and
(iii) detecting the amount of glucose present in the hydrogel patch.

**Patentansprüche**

1. Hydrogelpflaster, umfassend:

(a) eine hydrophile Verbindung, welche in Gegenwart von Wasser ein Hydrogel bildet, wobei die Verbindung in einer Menge von 4 Gew.-% oder mehr, bezogen auf das Gewicht des Hydrogels, anwesend ist;
(b) Wasser in einer Menge von 95% oder weniger, bezogen auf das Gewicht des Hydrogels;
(c) einen Elektrolyten, wobei das elektrische Hintergrundssignal in dem Gel kleiner als etwa 200 nA ist; und
(d) Glucoseoxidase, welche in einer Menge von 10 bis 5000 Einheiten pro Gramm des Gewichts des Hydrogels anwesend ist, wobei (i) die Glucoseoxidase eine zur Erzeugung von Wasserstoffperoxid führende Reaktion zwischen Glucose und Sauerstoff katalysieren kann, und (ii) Wasserstoffperoxid zersetzende Komponenten der Enzymzusammensetzung reduziert sind, so dass die quantitative Bestimmung des durch die Glucoseoxidasereaktion produzierten Wasserstoffperoxid nicht gestört ist;
wobei das Hydrogel innerhalb eines pH-Bereiches von 3 bis 9 gehalten wird.

2. Hydrogelpflaster gemäß Anspruch 1, wobei das elektrische Hintergrundssignal in dem Gel kleiner als etwa 50 nA ist.

3. Hydrogelpflaster gemäß einem vorstehenden Anspruch, wobei ein Produkt in der Reaktion in Schritt (d) nicht mehr als zu 20% in 30 Minuten abgebaut wird.

4. Hydrogelpflaster gemäß einem vorstehenden Anspruch, wobei die Diffusion eines Analyten, der in der Reaktion im Schritt (d) reagiert, geschwindigkeitsbestimmend ist und wobei die Diffusion den Analyten schneller als die Messzeit ist.

5. Hydrogelpflaster gemäß einem vorstehenden Anspruch, wobei das Hydrogel die durch die Formel:

1

$$L(K_c E / K_m D)^{\frac{1}{2}} \geq 1$$

definierte Eigenschaft aufweist,

wobei L die Hydrogeldicke ist, D die Diffusionskonstante eines Analyten ist, welcher in das Hydrogel hineingezogen wird, E die Enzymbeladung des Hydrogels ist, $K_c$ die katalytische Geschwindigkeitskonstante des Enzyms ist und $K_m$ die Michaelis-Menten-Geschwindigkeitskonstante des Enzyms ist.

6. Hydrogelpflaster gemäß einem vorstehenden Anspruch, wobei das Hydrogel weiterhin Bestandteile zur Beibehaltung eines ausgewählten Hydrogelmilieus umfaßt, und wobei das Milieu die Umwandlung des Analyten zum Produkt in der Reaktion des Schritts (d) verstärkt.

7. Hydrogelpflaster gemäß einem vorstehenden Anspruch, wobei das Enzym eine zur Erzeugung von Elektronen führende Reaktion zwischen Glucose und Sauerstoff katalysiert.

8. Hydrogelpflaster gemäß einem vorstehenden Anspruch, wobei das Enzym eine rekombinate oder synthetische Glucoseoxidase ist.

9. Hydrogelpflaster gemäß einem vorstehenden Anspruch, wobei das Enzym in einer Menge von 200 Einheiten oder mehr des Enzyms pro Gramm des Gewichts des Hydrogels anwesend ist.

10. Hydrogelpflaster gemäß einem vorstehenden Anspruch, welches weiterhin ein Mutarotase-Enzym umfasst.

11. Hydrogelpflaster gemäß einem vorstehenden Anspruch, weiterhin umfassend:

(e) ein Puffermittel, welches in einer Menge anwesend ist, die ausreicht, um einen pH-Wert im Bereich von 3 bis 9 in dem Hydrogel aufrechtzuerhalten.

12. Hydrogelpflaster gemäß Anspruch 11, wobei das Puffermittel in einer Menge anwesend ist, die ausreicht, um den pH-Wert des Hydrogels in einem Bereich von 6 bis 8 zu halten.

13. Hydrogelpflaster gemäß Anspruch 11 oder 12, wobei das Puffermittel in einer Menge anwesend ist, die ausreicht, um den pH-Wert des Hydrogels bei 7,4 zu halten.

14. Hydrogelpflaster gemäß Anspruch 11, 12 oder 13, wobei das Puffermittel einen Phosphatpuffer umfasst.

15. Hydrogelpflaster gemäß einem vorstehenden Anspruch, wobei die hydrophile Verbindung aus einem oder mehreren aus Polyethylenoxid, Polyvinylalkohol, Polyacrylsäure und Polyacrylamidomethylpropansulfonat und Copolymeren davon ausgewählt ist.

16. Hydrogelpflaster gemäß einem vorstehenden Anspruch, wobei die hydrophile Verbindung weiterhin Bisacrylamid umfasst.

17. Hydrogelpflaster gemäß einem vorstehenden Anspruch, wobei der Elektrolyt ein Chloridsalz ist.

18. Hydrogelpflaster gemäß einem vorstehenden Anspruch, welches weiterhin ein Biozid umfasst.

19. Hydrogelpflaster gemäß Anspruch 18, wobei das Biozid ein antibakterielles Mittel ist.

20. Hydrogelpflaster gemäß Anspruch 18, wobei das Biozid ein antimykotisches Mittel ist.

21. Hydrogelpflaster gemäß Anspruch 18, wobei das Biozid aus chlorierten Kohlenwasserstoffen, organometallischen Verbindungen, wasserstofffreisetzenden Verbindungen, Metallsalzen, organischen Schwefelverbindungen, quartären Ammoniumverbindungen, phenolischen Verbindungen und Methylparabenen ausgewählt ist.

22. Hydrogelpflaster gemäß Anspruch 21, wobei das Biozidmittel ein Methylparaben ist.

23. Hydrogelpflaster gemäß einem vorstehenden Anspruch, welches weiterhin ein Feuchthaltemittel umfasst.

24. Hydrogelpflaster gemäß Anspruch 23, wobei die hydrophile Verbindung in einer Menge im Bereich von 8% bis 12%, bezogen auf das Gewicht des das Feuchtmittel enthaltenden Hydrogels, anwesend ist.

25. Hydrogelpflaster gemäß einem vorstehenden Anspruch, wobei die hydrophile Verbindung aus einem oder mehreren aus Polyethylenoxid, Polyvinylalkohol, Polyacrylsäure und Polyacrylamidomethylpropansulfonat und Copolymeren davon ausgewählt ist,
wobei der Elektrolyt NaCl oder KCl ist,
wobei das Enzym Glucoseoxidase ist und die Glucoseoxidase in einer Menge in einem Bereich von 10 bis 5000 Einheiten pro Gramm der Summe von Bestandteil (a) und Bestandteil (b) anwesend ist, und
welches weiterhin ein Puffermittel umfasst, welches in dem Wasser gelöst ist, wobei das Puffermittel in einer Menge anwesend ist, die ausreicht, um den pH-Wert des Hydrogels im Bereich von 3 bis 9 zu halten.

26. Hydrogelpflaster, bestehend im Wesentlichen aus:

(a) einer hydrophilen Verbindung, welche ein Gel erzeugt, die in einer Menge im Bereich von 0,5% bis 40%, bezogen auf das Gewicht des Hydrogels, wenn auch ein Feuchthaltemittel zugesetzt ist, oder die in einer Menge von 15% bis 20%, bezogen auf das Gewicht des Hydrogels, wenn kein Feuchthaltemittel zugesetzt ist, anwesend ist;
(b) Wasser in einer Menge von 95% oder weniger, bezogen auf das Gewicht des Hydrogels;
(c) einem Elektrolyten, wobei das elektrische Hintergrundssignal in dem Gel kleiner als etwa 200 nA ist; und
(d) Glucoseoxidase, welche in einer Menge von 10 bis 5000 Einheiten pro Gramm des Gewichts des Hydrogels anwesend ist, wobei (i) die Glucoseoxidase eine zur Erzeugung von Wasserstoffperoxid führende Reaktion zwischen Glucose und Sauerstoff katalysieren kann, und (ii) Wasserstoffperoxid zersetzende Komponenten der Enzymzusammensetzung reduziert sind, so dass die quantitative Bestimmung des durch die Glucoseoxidasereaktion produzierten Wasserstoffperoxids nicht gestört ist.

27. Hydrogelpflaster gemäß Anspruch 1, wobei (a) die hydrophile Verbindung Polyethylenoxid umfasst, (b) das Wasser ein Puffermittel umfasst, und das Puffermittel ein Phosphatpuffer ist, und (c) der Elektrolyt Natriumchlorid umfasst.

28. Hydrogelpflaster gemäß einem vorstehenden Anspruch, wobei die hydrophile Verbindung in einer Menge von weniger als 40 Gew.-% anwesend ist und das Wasser in einer Menge von mehr als 60 Gew.-%, bezogen auf das Gewicht des Hydrogels, anwesend ist.

29. Hydrogelpflaster gemäß einem vorstehenden Anspruch, welches durch eine flache Gestaltung mit einer Dicke in einem Bereich von 5 μm bis 1,52 mm (60 mils) gekennzeichnet ist.

30. Hydrogelpflaster gemäß Anspruch 29, welches durch einer erste und eine zweite Oberfläche gekennzeichnet ist, wobei jede Oberfläche in einem Bereich von 0,5 cm$^2$ bis 10 cm$^2$ ist und wobei das Hydrogelpflaster eine Dicke von 5 μm bis 0,25 mm (10 mils) aufweist.

31. Hydrogelpflaster gemäß einem der Ansprüche 1 bis 28, wobei das Hydrogelpflaster eine Dicke von etwa 0,13 mm (5 mils) und eine Oberfläche von etwa 1 cm$^2$ aufweist.

32. Hydrogelpflaster gemäß einem der Ansprüche 1 bis 28, wobei das Hydrogelpflaster eine kreisförmige Scheibe ist, welche eine Dicke von 0,25 mm (10 mils) bis 1 mm (40 mils) und einen Durchmesser von 1 cm bis 3 cm aufweist.

33. Hydrogelpflaster gemäß Anspruch 32, wobei das Hydrogelpflaster eine Dicke von 1 mm (40 mils) und einen Durchmesser von 1,9 cm (0,75 Zoll) aufweist.

34. Hydrogelpflaster gemäß Anspruch 32, wobei das Hydrogelpflaster eine Dicke von 0.64 mm (25 mils) und einen Durchmesser von 1 cm aufweist.

35. Hydrogelpflaster gemäß einem vorstehenden Anspruch, welches weiterhin ein strukturierendes Trägermaterial, eingelagert in dem Hydrogelpflaster, umfasst.

36. Hydrogelpflaster gemäß Anspruch 35, wobei das strukturierende Trägermaterial ein Vliesgewebe ist, welches eine Außenparametergestalt und Größe aufweist, die im Wesentlichen gleich dem des Hydrogelpflasters ist.

**37.** Hydrogelpflaster gemäß einem vorstehenden Anspruch, wobei das Hydrogelpflaster eine erste und zweite Oberflächen aufweist, wobei das Hydrogelpflaster weiterhin erste und zweite, jeweils auf der ersten Oberfläche und der zweiten Oberfläche angebrachte Trennmittelschichten und ein in dem Material, welches das Wasser am Platz hält, eingelagertes Vliesmaterial umfasst.

**38.** Hydrogelpflaster gemäß einem vorstehenden Anspruch, wobei das Hydrogelpflaster in Gestalt einer dünnen, flachen Scheibe ist, welche sich den Konturen der menschlichen Haut anpassen und gegebenenfalls, darin eingelagert, ein Vliesgewebe oder eine poröse Membran aufweisen kann.

**39.** Hydrogelpflaster gemäß einem vorstehenden Anspruch, wobei ein oder mehrere Bestandteile des Gels behandelt wurden, um ein elektrisches Hintergrundssignal verursachende Verbindungen zu entfernen.

**40.** Hydrogelpflaster gemäß Anspruch 39, wobei eine oder mehrere der Gelbestandteile behandelt wurden, indem ein Diafiltrationsverfahren zur Entfernung elektroaktiver Verbindungen daraus verwendet wurde.

**41.** Verwendung des Hydrogelpflasters gemäß einem der Ansprüche 1 bis 40 zur Herstellung einer Vorrichtung zur Verwendung in einer Diagnosemethode für elektroosmotisches Extrahieren von Glucose durch die Haut eines Menschen und in das Hydrogelpflaster hinein, wobei die Diagnosemethode die Schritte umfasst:

(i) Aufbringen der das Hydrogelpflaster umfassenden Vorrichtung auf die Haut des Menschen, wobei das Hydrogelpflaster in Kontakt mit einer Elektrode steht, und
(ii) Erzeugen eines elektrischen Stroms, so dass sich die Glucose durch die Haut und in das Hydrogelpflaster hinein bewegt.

**42.** Verwendung des Hydrogelpflasters gemäß einem der Ansprüche 1 bis 40 zur Herstellung einer Vorrichtung zur Verwendung in einer Diagnosemethode für die Detektion einer Glucosemenge in einem Menschen, wobei die Diagnosemethode die Schritte umfasst:

(i) Extrahieren von Glucose durch die Haut des Menschen unter Verwendung der Vorrichtung, welche das mit einer Elektrode in Kontakt stehende Hydrogelpflaster umfasst,
(ii) Erzeugen eines elektrischen Stroms, so dass sich die Glucose durch die Haut und in das Hydrogelpflaster hinein bewegt, und
(iii) Detektieren der im Hydrogelpflaster anwesenden Glucosemenge.

## Revendications

**1.** Patch hydrogel comprenant :

(a) un composé hydrophile qui forme un hydrogel en présence d'eau, lequel composé est présent en une quantité de 4 % ou plus en poids, sur la base du poids de l'hydrogel ;
(b) de l'eau en une quantité de 95 % ou moins, sur la base du poids de l'hydrogel ;
(c) un électrolyte, dont le signal électrique de fond dans le gel est inférieur à environ 200 nA ; et
(d) de la glucose-oxydase présente en une quantité de 10 unités à 5 000 unités par gramme d'hydrogel, dans lequel (i) la glucose-oxydase peut catalyser une réaction entre le glucose et l'oxygène entraînant la production de peroxyde d'hydrogène, et (ii) les composants de dégradation du peroxyde d'hydrogène de la composition d'enzymes sont réduits, de telle sorte que la quantification du peroxyde d'hydrogène produit par la réaction de la glucose-oxydase ne soit pas compromise ;
dans lequel l'hydrogel est maintenu dans une plage de pH de 3 à 9.

**2.** Patch hydrogel selon la revendication 1, dans lequel ledit signal électrique de fond dans le gel est inférieur à environ 50 nA.

**3.** Patch hydrogel selon l'une quelconque des revendications précédentes, dans lequel un produit de la réaction de l'étape (d) n'est pas dégradé de plus de 20 % en 30 minutes.

**4.** Patch hydrogel selon l'une quelconque des revendications précédentes, dans lequel la diffusion d'un analyte réagissant dans la réaction de l'étape (d) limite la vitesse de réaction, et dans lequel la diffusion de l'analyte est plus rapide que le temps de mesure.

5. Patch hydrogel selon l'une quelconque des revendications précédentes, dans lequel l'hydrogel a la propriété définie par la formule :

$$L(K_c E/K_m D)^{\frac{1}{2}} \geq 1$$

où L est l'épaisseur de l'hydrogel, D est la constante de diffusion d'un analyte entraîné dans l'hydrogel, E est la charge en enzyme de l'hydrogel, $K_c$ est la constante catalytique de l'enzyme, et $K_m$ est la constante de Michaelis-Menten de l'enzyme.

6. Patch hydrogel selon l'une quelconque des revendications précédentes, dans lequel l'hydrogel comprend en outre des composants pour maintenir un environnement d'hydrogel particulier, et dans lequel l'environnement accroît la conversion de l'analyte en produit de la réaction de l'étape (d).

7. Patch hydrogel selon l'une quelconque des revendications précédentes, dans lequel l'enzyme catalyse une réaction entre le glucose et l'oxygène entraînant la production d'électrons.

8. Patch hydrogel selon l'une quelconque des revendications précédentes, dans lequel l'enzyme est une glucose-oxydase recombinante ou synthétique.

9. Patch hydrogel selon l'une quelconque des revendications précédentes, dans lequel l'enzyme est présente en une quantité de 200 unités ou plus d'enzyme par gramme d'hydrogel.

10. Patch hydrogel selon l'une quelconque des revendications précédentes, comprenant en outre une enzyme mutarotase.

11. Patch hydrogel selon l'une quelconque des revendications précédentes, comprenant en outre :

(e) un agent tampon présent en une quantité suffisante pour maintenir un pH dans l'hydrogel dans une plage de 3 à 9.

12. Patch hydrogel selon la revendication 11, dans lequel l'agent tampon est présent en une quantité suffisante pour maintenir le pH de l'hydrogel dans une plage de 6 à 8.

13. Patch hydrogel selon la revendication 11 ou 12, dans lequel l'agent tampon est présent en une quantité suffisante pour maintenir le pH de l'hydrogel à 7,4.

14. Patch hydrogel selon la revendication 11, 12 ou 13, dans lequel l'agent tampon comprend un tampon phosphate.

15. Patch hydrogel selon l'une quelconque des revendications précédentes, dans lequel le composé hydrophile est constitué d'un ou plusieurs éléments choisis dans le groupe constitué par l'oxyde de polyéthylène, l'alcool polyvinylique, l'acide polyacrylique et le polyacrylamidométhylpropanesulfonate, et les copolymères de ceux-ci.

16. Patch hydrogel selon l'une quelconque des revendications précédentes, dans lequel le composé hydrophile comprend en outre du bisacrylamide.

17. Patch hydrogel selon l'une quelconque des revendications précédentes, dans lequel l'électrolyte est un sel chlorure.

18. Patch hydrogel selon l'une quelconque des revendications précédentes, comprenant en outre un biocide.

19. Patch hydrogel selon la revendication 18, dans lequel le biocide est un agent antibactérien.

20. Patch hydrogel selon la revendication 18, dans lequel le biocide est un agent antifongique.

21. Patch hydrogel selon la revendication 18, dans lequel le biocide est choisi parmi des hydrocarbures chlorés, des organo-métalliques, des composés libérant de l'hydrogène, des sels métalliques, des composés de soufre organiques, des composés d'ammonium quaternaire, des dérivés phénoliques et des p-hydroxybenzoates de méthyle.

**22.** Patch hydrogel selon la revendication 21, dans lequel l'agent biocide est un p-hydroxybenzoate de méthyle.

**23.** Patch hydrogel selon l'une quelconque des revendications précédentes, comprenant en outre un humectant.

**24.** Patch hydrogel selon la revendication 23, dans lequel le composé hydrophile est présent en une quantité dans la plage de 8 % à 12 %, sur la base du poids de l'hydrogel contenant l'humectant.

**25.** Patch hydrogel selon l'une quelconque des revendications précédentes, dans lequel le composé hydrophile est constitué d'un ou plusieurs éléments choisis dans le groupe constitué par l'oxyde de polyéthylène, l'alcool polyvinylique, l'acide polyacrylique et le polyacrylamidométhylpropanesulfonate, et les copolymères de ceux-ci,
dans lequel l'électrolyte est NaCl ou KCl,
dans lequel l'enzyme est la glucose-oxydase et la glucose-oxydase est présente en une quantité dans la plage de 10 unités à 5 000 unités par gramme de la somme du composant (a) et du composant (b), et lequel comprend en outre un agent tampon dissous dans l'eau, lequel agent tampon est présent en une quantité suffisante pour maintenir le pH de l'hydrogel dans la plage de 3 à 9.

**26.** Patch hydrogel, constitué essentiellement de :

(a) un composé hydrophile, lequel forme un gel, présent en une quantité dans la plage de 0,5 % à 40 % sur la base du poids de l'hydrogel lorsqu'un humectant est également ajouté, ou présent en une quantité de 15 % à 20 % sur la base du poids de l'hydrogel lorsqu'aucun humectant n'est ajouté ;
(b) de l'eau en une quantité de 95 % ou moins sur la base du poids de l'hydrogel ;
(c) un électrolyte, dont le signal électrique de fond dans le gel est inférieur à environ 200 nA ; et
(d) de la glucose-oxydase présente en une quantité de 10 unités à 5 000 unités par gramme d'hydrogel, dans lequel (i) la glucose-oxydase peut catalyser une réaction entre le glucose et l'oxygène entraînant la production de peroxyde d'hydrogène, et (ii) les composants de dégradation du peroxyde d'hydrogène de la composition d'enzymes sont réduits, de telle sorte que la quantification du peroxyde d'hydrogène produit par la réaction de la glucose-oxydase ne soit pas compromise.

**27.** Patch hydrogel selon la revendication 1, dans lequel (a) le composé hydrophile comprend de l'oxyde de polyéthylène, (b) l'eau comprend un agent tampon et l'agent tampon est un tampon phosphate, et (c) l'électrolyte comprend du chlorure de sodium.

**28.** Patch hydrogel selon l'une quelconque des revendications précédentes, dans lequel le composé hydrophile est présent en une quantité inférieure à 40 % en poids et l'eau est présente en une quantité supérieure à 60 % en poids, sur la base du poids de l'hydrogel.

**29.** Patch hydrogel selon l'une quelconque des revendications précédentes, **caractérisé par** une configuration plate ayant une épaisseur dans la plage de 5 μm à 1,52 mm (60 mils).

**30.** Patch hydrogel selon la revendication 29, **caractérisé par** une première et une seconde surfaces spécifiques, chaque surface spécifique étant dans une plage de 0,5 cm$^2$ à 10 cm$^2$, lequel patch hydrogel a une épaisseur de 5 μm à 0,25 mm (10 mils).

**31.** Patch hydrogel selon l'une quelconque des revendications 1 à 28, lequel patch hydrogel a une épaisseur d'environ 0,13 mm (5 mils) et une surface spécifique d'environ 1 cm$^2$.

**32.** Patch hydrogel selon l'une quelconque des revendications 1 à 28, lequel patch hydrogel est un disque circulaire ayant une épaisseur de 0,25 mm (10 mils) à 1 mm (40 mils) et un diamètre de 1 cm à 3 cm.

**33.** Patch hydrogel selon la revendication 32, lequel patch hydrogel a une épaisseur de 1 mm (40 mils) et un diamètre de 1,9 cm (0,75 pouce).

**34.** Patch hydrogel selon la revendication 32, lequel patch hydrogel a une épaisseur de 0,64 mm (25 mils) et un diamètre de 1cm.

**35.** Patch hydrogel selon l'une quelconque des revendications précédentes, comprenant en outre un matériau de support structurel intégré dans le patch hydrogel.

**36.** Patch hydrogel selon la revendication 35, dans lequel le matériau de support structurel est un textile non tissé ayant une configuration externe et une taille substantiellement identiques à celles du patch hydrogel.

**37.** Patch hydrogel selon l'une quelconque des revendications précédentes, lequel patch hydrogel a des première et seconde surfaces, ledit patch hydrogel comprenant en outre des première et seconde pellicules anti-adhésives disposées respectivement sur la première surface et la seconde surface, et un matériau non tissé intégré dans le matériau qui maintient l'eau en place.

**38.** Patch hydrogel selon l'une quelconque des revendications précédentes, lequel patch hydrogel se présente sous la forme d'un disque mince et plat qui épouse les contours de la peau humaine et dans lequel peut facultativement être intégré un textile non tissé ou une membrane poreuse.

**39.** Patch hydrogel selon l'une quelconque des revendications précédentes, dans lequel un ou plusieurs composants du gel ont été traités afin d'extraire les composés pouvant occasionner un signal électrique de fond.

**40.** Patch hydrogel selon la revendication 39, dans lequel un ou plusieurs des composants dudit gel ont été traités par une procédure de diafiltration pour en extraire les composés électroactifs.

**41.** Utilisation du patch hydrogel selon l'une quelconque des revendications 1 à 40 pour la fabrication d'un dispositif destiné à être utilisé dans une méthode diagnostique pour extraire par électroosmose le glucose à travers la peau d'un être humain et l'amener dans ledit patch hydrogel, dans laquelle ladite méthode diagnostique comprend les étapes consistant à :

(i) appliquer le dispositif comprenant le patch hydrogel sur la peau de l'être humain, ledit patch hydrogel étant en contact avec une électrode, et
(ii) créer un courant électrique qui amène le glucose dans le patch hydrogel à travers la peau.

**42.** Utilisation du patch hydrogel selon l'une quelconque des revendications 1 à 40 pour la fabrication d'un dispositif destiné à être utilisé dans une méthode diagnostique pour détecter une quantité de glucose chez un être humain, dans laquelle ladite méthode diagnostique comprend les étapes consistant à :

(i) extraire le glucose à travers la peau de l'être humain en utilisant le dispositif comprenant le patch hydrogel en contact avec une électrode,
(ii) créer un courant électrique qui amène le glucose dans le patch hydrogel à travers la peau, et
(iii) détecter la quantité de glucose présente dans le patch hydrogel.

**FIG. 1**

**FIG. 2**

**FIG. 3**

FIG. 4

SENSOR
SIGNAL

DIFFUSION-CONTROLLED
REGION

KINETIC-CONTROLLED
REGION

ENZYME CONCENTRATION

**FIG. 5**

EP 0 840 597 B1